(19) Europäisches Patentamt
European Patent Office
Office européen des brevets

(11) Publication number: **0 428 700 B1**

(12) **EUROPEAN PATENT SPECIFICATION**

(45) Date of publication of patent specification: **04.05.94**

(51) Int. Cl.5: **C07K 7/06**, A61K 37/02

(21) Application number: **90909646.3**

(22) Date of filing: **12.06.90**

(86) International application number:
**PCT/EP90/00922**

(87) International publication number:
**WO 90/15819 (27.12.90 90/29)**

# (54) IRREVERSIBLE BOMBESIN ANTAGONISTS.

(30) Priority: **15.06.89 GB 8913844**

(43) Date of publication of application:
**29.05.91 Bulletin 91/22**

(45) Publication of the grant of the patent:
**04.05.94 Bulletin 94/18**

(84) Designated Contracting States:
**AT BE CH DE DK ES FR GB IT LI LU NL SE**

(56) References cited:
**US-A- 4 331 661**
**US-A- 4 423 236**

**CANCER RESEARCH, vol. 35, March 1975, Baltimore, MD (US); D.J. REED et al., pp. 568-576&NUM;**

**BRITISH JOURNAL OF PHARMACOLOGY, vol. 55, October 1975, Basingstoke, Hants (GB); M. BROCCARDO et al., pp. 221-227&NUM;**

(73) Proprietor: **FARMITALIA CARLO ERBA S.r.l.**
**Via Carlo Imbonati 24**
**I-20159 Milano(IT)**

(72) Inventor: **DE CASTIGLIONE, Roberto**
**Via Domenichino, 38**
**I-20149 Milan(IT)**
Inventor: **GOZZINI, Luigia**
**Viale Stelvio, 27/4**
**I-20159 Milan(IT)**
Inventor: **LUCIETTO, Pierluigi**
**Via Teodosio n. 19**
**I-20131 Milan(IT)**
Inventor: **CORRADI, Fabio**
**Via Marchiondi, 7**
**I-20122 Milan(IT)**
Inventor: **CIOMEI, Marina**
**Via del Molo, 1**
**I-27020 Torre d'Isola(IT)**
Inventor: **MOLINARI, Isabella**
**Via Elba, 28**
**I-20144 Milan(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

PEPTIDES - CHEMISTRY, STRUCTURE & BIOLOGY, Proceedings of the 11th American Peptide Symposium, 09-14 July 1989, La Jolla, CA (US), edited by J.E. Rivier et al., ESCOM, Leiden (NL); R. de CASTIGLIONE et al., pp. 168-170&NUM;

(74) Representative: **Kolb, Helga, Dr. Dipl.-Chem. et al**
**Hoffmann, Eitle & Partner,**
**Patentanwälte,**
**Postfach 81 04 20**
**D-81904 München (DE)**

## Description

The present invention relates to peptide derivatives, to pharmaceutical compositions containing them, to processes for their preparation, and to their application as therapeutic agents.

In this specification symbols and abbreviations are those commonly used in peptide chemistry (see Eur.J. Biochem. (1984) 138, 9-37). Consequently, the three-letter amino acid symbols denote the L configuration of chiral amino acids. D-amino acids are represented by small letters: e.g., ala = D-Ala. Other symbols and abbreviations used are: AA, amino acid; AcOEt, ethylacetate; BBS, bombesin; Boc, t-butoxycarbonyl; BuOH, n-butyl alcohol; BOP, benzotriazolyloxy-tris[dimethylamino]phosphonium hexafluorophosphate; DCC, N,N'-dicyclohexylcarbodiimide; DMF, dimethylformamide; DMSO, dimethylsulfoxide; Dnp, 2,4-dinitrophenyl; EGF, epidermal growth factor; EtOH, ethyl alcohol; FAB (or FD)-MS, fast atom bombardment (or field desorption) mass spectrometry; ECC, ethylchlorocarbonate; $Et_2O$, diethylether; Glp, L-pyroglutamic acid; h-GRP (or p-GRP), human (or porcine) gastrin releasing peptide; HOBt, 1-hydroxybenzotriazole; I.D., internal diameter; MeOH, methyl alcohol; m.p., melting point; n.d., not determined; Nle, L-norleucine; NMM, N-methylmorpholine; NMR, nuclear magnetic resonance; OSu, N-hydroxysuccinimidyl; PE, petroleum ether 40°-70°; RP-HPLC, reversed phase high performance liquid chromatography; SCLC, small cell lung carcinoma; TFA, trifluoroacetic acid; THF, tetrahydrofuran; TLC, thin layer chromatography; Tos, p-toluensulphonyl.

The invention provides a peptide of the formula I:

R—A—B—C—Trp—Ala—Val—X—Y—T—W I

wherein

- R represents a group of the formula $4\text{-}(ClCH_2CH_2)_2N\text{-}C_6H_4\text{-}CO\text{-}$, $3\text{-}(ClCH_2CH_2)_2N\text{-}C_6H_4\text{-}CO\text{-}$, $ClCH_2CH_2NHCO\text{-}$, ClCH=CH-CO-, BrCH=CH-CO-, $CH_2$=CClCO-, $CH_2$=CBrCO- (either cis or trans isomers),

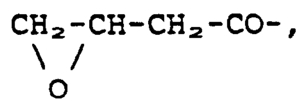

  CH ≡ C-CO-, $ClCH_2CH_2N(NO)CO\text{-}$, or $ClCH_2CO\text{-}CH(R_1)NHCO(CH_2)_2CO\text{-}$;
- A represents a valence bond, or a Gly, Leu-Gly, Arg-Leu-Gly, Gln-Arg-Leu-Gly residue,
- B represents a valence bond or a Asn or Thr residue;
- C represents a Gln or His residue,
- X represents a Gly or ala residue;
- Y represents a valence bond or a His($R_2$), his($R_2$), Phe, phe, Ser, ser, Ala or ala residue;
- T represents a valence bond or a Leu, leu, Phe or phe residue;
- W represents a group of the formula OH, $NH_2$, $NH(CH_2)_4CH_3$, $NH(CH_2)_2C_6H_5$, Met-$R_3$, Leu-$R_3$, Ile-$R_3$, or Nle-$R_3$,
- $R_1$ represents hydrogen atom, linear or branched aliphatic chain having for 1 to 11 carbon atoms, benzyl or phenyl group,
- $R_2$ represents hydrogen atom or Tos, Dnp or Bzl group and
- $R_3$ represents an amino, hydroxy, methoxy or hydrazino group.

Salts of these peptides with pharmaceutically acceptable acids are within the scope of the invention. Such acid addition salts can be derived from a variety of inorganic and organic acids such as sulfuric, phosphoric, hydrochloric, hydrobromic, hydroiodic, nitric, sulfamic, citric, lactic, pyruvic, oxalic, maleic, succinic, tartaric, cinnamic, acetic, trifluoracetic, benzoic, salicylic, gluconic, ascorbic and related acids.

Preferred alyphatic chains which $R_1$ may represent include methyl, ethyl, n-propyl, iso-propyl, n-butyl and iso-butyl groups.

The synthesis of the peptides of the invention may be accomplished by classical solution methods. The synthesis consists essentially of appropriate successive condensations of protected amino acids or peptides. The condensations are carried out so that the resulting peptides have the desired sequence of amino acid residues.

The amino acids and peptides, which can be condensed according to methods known in peptide chemistry, have the amino and carboxyl groups, not involved in peptide bond formation, blocked by suitable protecting groups capable of being removed by acid or alkali treatment or by hydrogenolysis.

For the protection of the amino group the following protective groups may, for example, be employed:

benzyloxycarbonyl, t-butoxycarbonyl, trityl, formyl, trifluoracetyl, o-nitrophenylsulphenyl, 4-methyloxybenzyloxycarbonyl, 9-fluorenylmethoxycarbonyl, 3,5-dimethoxy-α-α'-dimethylbenzyloxycarbonyl or methylsulphonylethoxycarbonyl.

For the protection of the carboxyl group the following protective groups may, for example, be employed: methyl, ethyl, t-butyl, benzyl, p-nitrobenzyl or fluorenylmethyl, amide, hydrazide, t-butoxycarbonyl hydrazide or benzyloxycarbonyl hydrazide.

The hydroxy functions of hydroxy amino acids and the imino function of histidine may be protected by suitable protecting groups (throughout all the synthesis or only during a few steps) or may be unprotected. For the protection of the hydroxy function the following protective groups may, for example, be employed; t-butyl, benzyl, acetyl. For the protection of the imidazole imino function the following groups may, for example, be used: 2,4-dinitrophenyl, tosyl, benzyl. De-protecting reactions are carried out according to methods known per se in peptide chemistry.

The condensation between an amino group of one molecule and a carboxyl group of another molecule to form the peptides linkage may be carried out through an activated acyl-derivative such as a mixed anhydride, an azide or an activated ester, or by direct condensation between a free amino group and a free carboxyl group, in the presence of a condensing agent such as dicyclohexylcarbodiimide, alone or together with a racemization preventing agent, such as N-hydroxysuccinimide or 1-hydroxybenzotriazole, or together with an activating agent such as 4-dimethylamino-pyridine. The condensation may be carried out in a solvent such as dimethylformamide, dimethylacetamide, pyridine, acetonitrile, tetrahydrofuran or N-methyl-2-pyrrolidone.

The reaction temperature may be from -30 °C to room temperature. The reaction time is generally from 1 to 120 hours.

The scheme of synthesis, the protecting groups and condensing agents are selected so as to avoid the risk of racemization.

## Biological activity

The peptides of the present invention are endowed with potent antagonism versus "in vitro" and "in vivo" effects induced by bombesin, such as contraction of smooth musculature, modification of behaviour of central origin and mitogenesis.

Bombesin (BBS) is a tetradecapeptide of formula Glp-Gln-Arg-Leu-Gly-Asn-Gln-Trp-Ala-Val-Gly-His-Leu-Met-$NH_2$, originally isolated from the skin of a frog. The biological activity resides in the C-terminal part of the molecule: BBS(6-14)nonapeptide is as active as the parent compound. The human counterpart of bombesin is a 27 amino acid peptide, known as gastrin-releasing peptide (h-GRP). Bombesin and bombesin-like peptides display a number of biological activities (J.H. Walsh (1983) in "Brain Peptides", D.T. Krieger, M.J. Brownstein and J.B. Martin (eds), Wiley Interscience Publ., pp. 941-960), including autocrine growth-promoting effects on human small cell lung carcinoma (SCLC) (F. Cuttitta et al. (1985) Cancer Survey, 4, 707-727), autocrine and/or paracrine stimulation of human prostatic cancer cell proliferation (M. Bologna et al., Cancer, in press) and modulation of the EGF receptor (I. Zachary and E. Rozengurt (1985) Cancer Surveys, 4, 729-765).

A bombesin antagonist, by competing with the natural ligand for the receptor(s), would inhibit the triggering of the cascade of events leading to abnormal cell proliferation.

Different approaches in this direction have been followed by different research groups. A series of C-terminal bombesin nona- and decapeptides, characterized by amino acid deletion, inversion or substitution, has been the object of a previous patent application by our side (EP Patent Application n° 0339193). These peptides, however, like other BBS antagonists, usually show moderate affinity for the BBS receptor.

The compounds of the present invention, having an alkylating moiety behave as receptor antagonists either when given in combination with bombesin or when administered 24 hours before bombesin challenge.

## Biological test results

The binding affinity of the compounds of the present invention for the bombesin receptors was determined on mouse Swiss 3T3 fibroblasts (I. Zachary and E. Rozengurt (1985) Proc. Natl. Acad. Sci. USA, 82, 7616-7620) (Table 1).

The effect on mitogenesis was determined in quiescent and confluent Swiss 3T3 cells maintained in serum free medium (A.N.Corps et al (1985) Biochem J. 231, 781-785). In a first set of experiments, analogues are given alone or in combination with bombesin. In a second set of experiments, cells are pre-treated with the alkylating peptides, washed, left at 37 °C for 24 hours and then challenged with bombesin. In both cases,

DNA synthesis was evaluated as [$H^3$]thymidine incorporation (Table 2).

Mitogenic effect of bombesin and its analogues were also evaluated as activation of the protein-tyrosin kinase that phosphorylates a 115 KD protein (p115) associated with the bombesin receptor complex (D. Cirillo et al. (1986) Mol.Cell. Biol. 6, 4641- 4649) (Table 3). In addition, exposure to these peptides in the 0.1-50 μM range was associated with significant reduction in the growth of SCLC cell lines (such as NCI-H345, NCI-N592, NCI-H128), as well as of prostatic carcinoma cell lines (such as DU145 and PC3).

Parenteral administration of these peptides at doses ranging between 1 ng/kg - 100 mg/kg to nude mice was associated with significant growth reduction of the above mentioned transplanted human SCLC and prostatic carcinoma cell lines.

TABLE 1

| BINDING AFFINITY OF BOMBESIN ALKYLATING ANALOGUES ON MOUSE SWISS 3T3 FIBROBLASTS | |
|---|---|
| COMPOUND | $ID_{50}$ (nM) |
| I | 1363 ± 266 |
| II | 438 ± 111 |
| III | 1815 ± 330 |
| IV | 0.7 ± 0.2 |
| V | 1336 ± 199 |
| VI | 648 ± 290 |
| Reference peptides: | |
| BBS | 12.6 ± 3.8 |
| Spantide | 11100 |
| [$pro^2$]Spantide | 14000 |
| [$Leu^{13}\psi(CH_2$-NH)$Leu^{14}$]BBS | 214 ± 30 |

TABLE 2

## $[H^3]$THYMIDINE INCORPORATION IN MOUSE SWISS 3T3 FIBROBLASTS

| COMPOUND | FOLD INCREASE OVER BASAL VALUE | | | | % INHIBITION IN THE PRESENCE OF 25nM BBS | | | |
|---|---|---|---|---|---|---|---|---|
| | | | | | A | | B | |
| | 5nM | 50nM | 0.5μM | 5 μM | 0.5μM | 5 μM | 0.5μM | 5 μM |
| I | N.D. | N.D. | 0.6 | 0.6 | 9 ± 6 | 12±3 | 0 | |
| II | N.D. | N.D. | 1.0 | 1.0 | 0 | 50±10 | 0 | |
| III | N.D. | N.D. | 2.3 | 3.4 | 0 | 0 | 51±11 | 53+8 |
| IV | N.D. | N.D. | 3.2 | 2.2 | 13±8 | 32±9 | 0 | 20±12 |
| V | N.D. | 1.8. | 1.8 | 2.5 | 0 | 0 | 0 | 0 |
| VI | N.D. | 1.0 | 1.0 | 1.0 | 0 | 0 | 39±14 | 29±10 |
| Reference peptides: | | | | | | | | |
| BBS | 3.0±1 | | | | | | | |
| $[Leu^{13}\psi(CH_2\text{-}NH)Leu^{14}]$BBS | | | 1 | 1 | 29±10 | 56±4 | 0 | 0 |

A= analogues are given in combination with BBS

B= cells are pre-treated with analogues, washed, left at 37°C for 24 h and then challenged with BBS

TABLE 3

| PHOSPHORYLATION OF THE p115 PROTEIN ASSOCIATED WITH THE BOMBESIN RECEPTOR | |
|---|---|
| COMPOUND | MINIMAL ACTIVE DOSE (nM) |
| I | > 4000 |
| II | > 1000 |
| III | 500 |
| IV | 500 |
| V | > 2000 |
| VI | > 1000 |
| Reference peptides: | |
| BBS | 3 |
| Spantide | > 10000 |
| $[pro^2]$Spantide | > 10000 |
| $[Leu^{13}\psi(CH_2\text{-}NH)Leu^{14}]$BBS | > 200 |

The peptides of the formula I, therefore, find application in the therapy of human neoplasms which are modulated in their growth and progression by peptides of the GRP family, either directly or in concert with other growth factors.

In addition, these alkylating analogues can be used in the management of the clinical symptoms associates with these deseases and due to hypersecretion of GRP-like peptides.

The compounds of the invention can be administered by the usual routes, for example, parenterally, e.g. by intravenous injection or infusion, or by intramuscular, subcutaneous, intracavity and intranasal administration.

The dosage depends on the age, weight and condition of the patient and on the administration route.

On the basis of the "in vitro" and "in vivo" data in mice it can be estimated that the therapeutic doses in humans will be in the range 1 ng/kg - 100 mg/kg, once to 6 times daily.

The invention also provides pharmaceutical compositions containing a compound of formula (I) as the active substance, in association with one or more pharmaceutically acceptable excipients.

The pharmaceutical compositions of the invention are usually prepared following conventional methods and are administered in a pharmaceutically suitable form.

For instance, solutions for intravenous injection or infusion may contain as carrier, for example, sterile water or, preferably, they may be in the form of sterile aqueous isotonic saline solutions.

Suspensions or solutions for intramuscular injections may contain, together with the active compound, a pharmaceutically acceptable carrier, e.g., sterile water, olive oil, ethyl oleate, glycols (e.g., propylene glycol) and, if desired, a suitable amount of lidocaine hydrochloride.

Furthermore, according to the invention, there are provided pharmaceutical compositions for use in a method of treating neuroendocrine neoplasms (such as small cell lung carcinoma and prostatic carcinoma) or the clinical symptoms associated with these diseases in patients in need of it.

Chemistry

Methods:

a) TLC was performed on pre-coated plates of silica gel 60 $F_{254}$ (Merck), layer thickness 0.25 mm, length 20 cm, with the following eluents:

System A: n-butanol/acetic acid/water = 600/150/150 by volume
System B: chloroform/methanol/$NH_4OH$ 30% = 500/346 154 by volume
System C: chloroform/methanol = 90/10 by volume
System D: dichloromethane/diethylether = 90/10 by volume
System E: acetonitrile/water/formic acid = 80/20/10.

b) Analytical RP-HPLC was performed on a Hewlett Packard Mod. 1084 apparatus on a LiChrosorb Hibar RP-18 column (Merck) 250 x 4 mm I.D., particle diameter 5 µ. The following eluents were used:
A = $KH_2PO_4$ 20 mM, pH 3.5/acetonitrile 9/1 by volume
B = $KH_2PO_4$ 20 mM, pH 3.5/acetonitrile 3/7 by volume.

The elution is programmed with a linear gradient from 60% to 90% B over a period of 20 min (system A) or from 30 to 70% B over a period of 15 min (System B), and then isocratically for 15 min, with a flow rate of 1 ml/min.
The peptides are characterized by their retention time (RT).
c) Preparative RP-HPLC was performed using a Delta Prep 3000 apparatus (Waters) on a Deltapak column (Waters), 300 x 19 mm I.D., particle diameter, 10 $\mu$. The following eluents were used:
A = 0.05% TFA in water
B = 0.05% TFA in acetonitrile/water 7/3 by volume.
Flow rate = 24 ml/min; detection wavelength = 220 nm.
Elution methods are reported in the single examples.
In each case, fractions were checked by analytical RP-HPLC and those showing a purity greater than 98% were pooled. After removal of acetonitrile, the solution was lyophilized.
d) Amino acid analysis was carried out on acid hydrolysates (either at 110 °C for 22 h in 6 N HCl + 0.1% phenol or at 100 °C for 16 h in 3 N mercaptoethansulfonic acid, both under $N_2$). Only natural amino acid residues were determined. Due to partial decomposition in normal hydrolysis conditions, Trp was determined only in hydrolysates with mercaptoethansulfonic acid.

Example 1

Preparation of 4-$(ClCH_2CH_2)_2NC_6H_4CO$-Thr-Gln-Trp-Ala-Val-Gly-Leu-Met-$NH_2$ (I).

25.2 mg (0.096 mmol) of [p-bis(2-chloroethyl)amine]benzoic acid were dissolved in 5 ml of distilled DMF and 60 mg (0.064 mmol) of H-Thr-Gln-Trp-Ala-Val-Gly-Leu-Met-$NH_2$ . HCl (EP Patent Appl. N. 89102283.2) were successively added; the solution was cooled to 5 °C and 0.0176 ml of NMM (0.16 mmol) and 0.0425 g of BOP (0.096 mmol) were added. The reaction mixture was stirred at room temperature overnight, then it was poured drepwise into 100 ml of a 10% solution of citric acid at 5 °C. The mixture was stirred for 1 h at a temperature below 10 °C, then filtered and washed with water to neutrality: 66.1 mg of crude product (90% yield) were obtained. The product was purified by preparative RP-HPLC, running a gradient from 60% to 90% of eluent B in eluent A over 30 min (flow rate, 35 ml/min): 33 mg of product I (50% yield) were obtained: $Rf_A$ 0.61; $RT_A$ = 8.66; FAB-MS: m/z 1147 ($MH^+$); AA ratios: Thr 0.98 (1), Glu 1, Ala 0.97 (1), Val 0.95 (1), Gly 1.04 (1), Leu 1.02 (1), Met 0.90 (1) (Trp n.d.).

Example 2

Preparation of 4-$(ClC_2CH_2)_2NC_6H_4CO$-Thr-Gln-Trp-Ala-Val-Gly-Leu-Nle-$NH_2$ (II).

0.2 g (0.216 mmol) of H-Thr-Gln-Trp-Ala-Val-Gly-Leu-Nle-$NH_2$ . HCl (prepared following the methodology described in our EP Patent Appl. N. 89102283.2 for H-Thr-Gln-Trp-Ala-Val-Gly-Leu-Met-$NH_2$ . HCl) were reacted with 0.085 g (0.324 mmol) of [p-bis(2-chloroethyl) amine]benzoic acid, g 0.143 (0.324 mmol) of BOP and 0.059 ml of NMM (0.537 mmol) in 17 ml of distilled DMF, as described in example 1. The crude product was purified by preparative RP-HPLC, running a gradient from 45% to 90% of eluent B in eluent A over 20 min: 0.122 g (50% yield) of product II were obtained: $Rf_A$ 0.75; $RT_A$ 10.39; FAB-MS: m/z 1129 ($MH^+$); AA ratios: Thr 0.97 (1), Glu 1, Ala 0.94 (1), Val 0.96 (1), Gly (1), Leu 0.92 (1), Nle 0.87 (1) (Trp n.d.).

Example 3

Preparation of 4-$(ClCH_2CH_2)_2NC_6H_4CO$-Thr-Gln-Trp-Ala-Val-Gly-His(Dnp)-Leu-Met-$NH_2$ (III).

From 63 mg (0.24 mmol) of [p-bis(2-chloroethyl)amine]benzoic acid and 200 mg (0.16 mmol) of HCl . H-Thr-Gln-Trp-Ala-Val-Gly-His(Dnp)-Leu-Met-$NH_2$ [prepared from Boc-Thr-Gln-Trp-Ala-Val-Gly-OH (our EP Patent Application N. 89102283.2) and HCl . H-His(Dnp)-Leu-Met-$NH_2$ (F. Angelucci and R. de Castiglione (1975) Experientia, 507-508) following the procedure described in the same patent application for analogous compounds] and operating as described in Example 1, 161 mg (70%) yield) of crude compound III were obtained. The product was purified by preparative RP-HPLC running a gradient from 60% to 90% of eluent B in eluent A over 20 min: 60 mg (26% yield) of product III were obtained $Rf_A$ 0.70; $RT_A$ 14.45; $RT_B$ 24.82; FAB-MS : m/z 1451 ($MH^+$); AA ratios: Thr 1.05 (1), Glu 1, Gly 1.03 (1), Ala 0.99 (1), Val 0.95 (1), Met 0.90 (1), Leu 0.97 (1), (Trp and His n.d.)

Example 4

Preparation of 4-$(ClCH_2CH_2)_2NC_6H_4CO$-Thr-Gln-Trp-Ala-Val-Gly-His-Leu-Met-$NH_2$ (IV).

65 mg (0.05 mmol) of compound (III) were suspendend in 3 ml of 0.02 M phosphate buffer pH 8 and treated with of 2-mercapto-ethanol. The resulting clear solution was left to react for 20 min, then poured with stirring into 300 ml $Et_2O$. The precipitate was filtered, thoroughly washed with $Et_2O$, then distributed between Bu-OH and water. The organic phase was concentrated to small volume and the peptide precipitated by dilution with AcOEt/$Et_2O$: 50 mg of the crude compound (IV) (71% yield) were obtained. The product was purified by preparative RP-HPLC running a gradient from 40% to 90% of eluent B in eluent A over 30 min: 25 mg (36% yield) of IV were obtained : $Rf_A$ 0.39; $RT_A$ 9.26 $RT_A$ 9.26 ; $RT_B$ 19.41; FAB-MS : m/z 1284 ($MH^+$); AA ratios: Thr 1.05 (1); Gln (1), Gly 1.03 (1), Ala 0.99 (1), Val 0.95 (1), Met 0.88 (1), His 0.91 (1) (Trp n.d.).

Example 5

Preparation of $ClCH_2CH_2N(NO)CO$-Thr-Gln-Trp-Ala-Val-Gly-Leu-Nle-$NH_2$ (V).

Step 1

$ClCH_2CH_2NHCO$-OSu (Va)

5.75 g (0.05 mol) of N-hydroxysuccinimide were dissolved in 125 ml of AcOEt and made to react at 0 ° C with 12.92 ml (0.075 mmol) of N-ethyl-diisopropylamine and 5.12 ml (0.060 mol) of 2-chloroethylisocianate, added dropwise consecutively. The reaction mixture was left 48 hours under vigorous stirring, then the solvent was evaporated and the residue distributed between AcOEt and water. The organic layer was dried over $Na_2SO_4$ and evaporated to small volume. The product crystallized upon standing in the cold: 7.68 g (70% yield) of compound (Va) were obtained: m.p. 104-107 ° C; $Rf_C$ 0.43; FAB-MS: m/z 220 ($M^{+\bullet}$); $^1$H-NMR (200 MHz, DMSO-$d_6$) δ(ppm): 8.57 (t, 1H, $CH_2\underline{NH}CO$), 3.64 (t, 2 H, $Cl\underline{CH_2}CH_2$), 3.39 (m, 2 H, $CH_2\underline{CH_2}NH$), 2.75 (s, 4 H, succinimide); elemental analysis ($C_7H_9N_2O_4Cl$): C 38.10 (38.11), H 4.10 (4.11), N 12.67 (12.70), Cl 16.00 (16.07).

$ClCH_2CH_2N(NO)CO$-OSu (Vb)

1.100 g (0.005 mol) of compound (Va) were dissolved in 12 ml of $CH_2Cl_2$ and made to react at 0 ° C with 0.800 ml (0.010 mol) of pyridine and 14.0 ml (0.020 mol) of 1.4 N NOCl in $CH_2Cl_2$. The reaction mixture was left under vigorous stirring for five fours, then was washed with cold water, dried over $Na_2SO_4$ and evaporated to small volume. The crystallization began at room temperature and was completed in the cold. 0.80 g (63%) of compound (V b) were obtained: m.p. 101-103 ° C; $Rf_C$ 0.87; FAB-MS: m/z 249 ($M^{+\bullet}$); $^1$H-NMR (200 MHz, DMSO-$d_6$)δ (ppm): 4.13 (t, 2 H, $Cl\underline{CH_2}CH_2$), 3.69 (t, 2 H, $CH_2\underline{CH_2}NNO$), 2.89 (s, 4 H, succinimide); elemental analysis ($C_7H_8N_3O_5Cl$): C 33.70 (33.68), H 3.25 (3.23), N 16.82 (16.83), Cl 14.80 (14.20).

$ClCH_2CH_2N(NO)CO$-Thr-Gln-Trp-Ala-Val-Gly-Leu-Nle-$NH_2$ (V)

0.185 g (0.2 mmol) of HCl . H-Thr-Gln-Trp-Ala-Val-Gly-Leu-Nle-$NH_2$ (see example 2) were suspended in a mixture of 0.5 ml of hexamethylphosphorylamide and 0.5 ml of N-methylpyrrolidone and made to react at room temperature with 0.028 ml (0.2 mmol) of triethylamine followed by 0.100 g (0.4 mmol) of compound (Vb). After a 2 h stirring, the reaction mixture was diluted with water: 0.150 g (73% yield) of crude compound (V) were obtained. A 50 mg sample was purified by preparative RP-HPLC running a gradient from 10% to 90% eluent A in eluent B over 30 min: $Rf_B$ 0.59; $RT_B$ 16.10; FAB-MS: m/z 1020 ($MH^{+\bullet}$); $^1$H-NMR (200 MHz, DMSO-$d_6$)δ (ppm) i.a.: 3.60 (t, 2 H, $Cl\underline{CH_2}CH_2$); AA ratios: Thr 0.91 (1), Glu 1.07 (1), Ala 1.03 (1), Val 1, Gly 1.02 (1), Leu 1.01 (1), Nle 0.90 (1) (Trp n.d.).

Example 6

Preparation of $CH_3(CH_2)_3CH(COCH_2Cl)NHCO(CH_2)_2CO$-Thr-Gln-Trp-Ala-Val-Gly-Leu-Met-$NH_2$ (VI)

Step 1

Boc-Nle-CH = $N_2$ (VIa)

4.60 g (0.02 mol) of Boc-Nle-OH in 40 ml anhydrous THF were made to react at -12 °C with 2.20 ml (0.02 mol) of NMM and 2.62 ml (0.02 mol) of isobutylchlorocarbonate. After 1 min the reaction was quenched by adding 50 ml $Et_2O$ and cooling the solution to -35 °C. Salts were filtered off and the filtrate cooled to -70 °C.
In a separate vessel, 3.96 g (0.06 mol) of KOH dissolved in 20 ml of 50% EtOH were added dropwise in 10 min at 0 °C to a solution of 6.43 g (0.03 mol) of N-methyl-N-nitroso-p-toluensulfonamide in 100 ml $Et_2O$ and 10 ml ethylenglycol monomethylether. The so formed diazomethane was distilled directly into the reaction vessel containing the preformed and chilled mixed anhydride. After stirring the reaction mixture at 0 °C for 20 hours, the solvent was evaporated under reduced pressure and the residue distributed between AcOEt and 0.5 M aqueous $KHCO_3$. The organic layer was washed to neutrality with brine, dried over $Na_2SO_4$ and evaporated in vacuo to give an oil that crystallized upon standing in the cold: 2.9 g (57% yield) of compound (VIa) were obtained: m.p. 83-85 °C; $Rf_D$ 0.51; $^1$H-NMR (200 MHz, DMSO-$d_6$)$\delta$ (ppm) i.a.: 6.00 (s, 1 H, CH = $N_2$); FD-MS: m/z 255• ($M^{+\bullet}$); elemental analysis ($C_{12}H_{21}N_3O_3$): C 56.50 (56.45), H 8.30 (8.29), N 16.47 (16.46).

step 2

HCl . H-Nle-$CH_2Cl$ (VIb)

2.4 g (0.01 mol) of Boc-Nle-CH = $N_2$ (VIa) were dissolved in 20 ml of 4.4 N HCl in dioxane. After 5 min at room temperature the product was precipitated with $Et_2O$: 1.56 g (78% yield) of compound (VIb) were obtained: m.p. 153-154 °C; $Rf_E$ 0.63; $^1$H-NMR (200 MHz, DMSO-$d_6$) $\delta$ (ppm) i.a.: 4.84, 4.74 (two d, 2 H, $CH_2$-Cl); FD-MS: m/z 199 ($M^{+\bullet}$);elemental analysis ($C_7H_{15}NOCl_2$); C 42.02 (42.01), H 7.54 (7.55), N 6.96 (6.99), Cl 35.42 (35.43).

HOOC-$CH_2CH_2$-CO-Nle-$CH_2Cl$ (VIc)

0.60 g (3 mmol) of HCl . H-Nle-$CH_2Cl$ (VIb) and 0.33 ml (3 mmol) of NMM were dissolved in 10 ml of DMF and made to react at 0 °C with 0.30 g (3 mmol) of succinic anhydride in 5 ml of DMF added dropwise in 10 min. After 30 min the reaction mixture was allowed to reach room temperature and kept under stirring 4 h longer. The solvent was evaporated under reduced pressure, the residue taken up in AcOEt and, after filtration of the insoluble material, washed with 0.02 M HCl, brine, and finally dried over $Na_2SO_4$. Evaporation of the solvent and trituration with $Et_2O$/PE gave 0.35 g (44% yield) of compound (VIc): m.p. 114-116 °C; $Rf_B$ 0.33; $^1$H-NMR (200 MHz, DMSO-$d_6$) $\delta$ (ppm) i.a.: 4.57 (s, 2H, $CH_2$-Cl); elemental analysis ($C_{11}H_{18}NO_4Cl$): C 49.74 (50.10), H 5.34 (5.31), Cl 13.16 (13.44).

$CH_3(CH_2)_3CH(COCH_2Cl)NHCO(CH_2)_2CO$-Thr-Gln-Trp-Ala-Val-Gly-Leu-Met-$NH_2$ (VI)

100 mg (0.106 mmol) of HCl . H-Thr-Gln-Trp-Ala-Val-Gly-Leu-Met-$NH_2$ (EP Patent Appl. N. 89102283.2) and 0.006 ml (0.106 mmol) NMM in 2 ml DMF were made to react at 0 °C with 31 mg (0.120 mmol) HOOC-$(CH_2)_2$CO-Nle-$CH_2Cl$ (VIc), 19 mg (0.143 mmol) HOBT and 27 mg (0.133 mmol) DCC. The reaction mixture was then stirred for 4 days at room temperature. The solvent was evaporated under reduced pressure, the residue triturated with warm isopropyl alcohol, and the insoluble material isolated by filtration: 50 mg (40% yield) of compound (VI) were obtained: $Rf_A$ 0.42; $RT_B$ 15.10; FAB-MS: m/z 1149 ($MH^+$); AA ratios: Thr 0.95 (1), Glu 1, Ala 1.08 (1), Val 0.97 (1), Gly 1.01 (1), Leu 1.00 (1), Met 0.93 (1) (Trp n.d.).

## Claims

### Claims for the following Contracting States : AT, BE, CH, DE, DK, FR, GB, IT, LI, LU, NL, SE

**1.** A peptide of the formula I:

R—A—B—C—Trp—Ala—Val—X—Y—T—W I

wherein R represents a group of the formula $4\text{-}(ClCH_2CH_2)_2N\text{-}C_6H_4\text{-}CO\text{-}$, $3\text{-}(ClCH_2CH_2)_2N\text{-}C_6H_4\text{-}CO\text{-}$, $ClCH_2CH_2NHCO\text{-}$, $ClCH=CH\text{-}CO\text{-}$, $BrCH=CH\text{-}CO\text{-}$, $CH_2=CClCO\text{-}$, $CH_2=CBrCO\text{-}$(either cis or trans isomers),

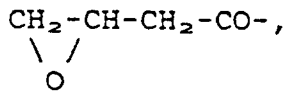

$HC\equiv C\text{-}CO\text{-}$, $ClCH_2CH_2N(NO)CO\text{-}$, or $ClCH_2CO\text{-}CH(R_1)NHCO(CH_2)_2CO\text{-}$;

- A represents a valence bond, or a Gly, Leu-Gly, Arg-Leu-Gly, Gln-Arg-Leu-Gly residue;
- B represents a valence bond or a Asn or Thr residue;
- C represents a Gln or His residue;
- X represents a Gly or ala residue;
- Y represents a valence bond or a His($R_2$), his($R_2$), Phe, phe, Ser, ser, Ala or ala residue;
- T represents a valence bond or a Leu, leu, Phe or phe residue;
- W represents a group of the formula OH, $NH_2$, $NH(CH_2)_4CH_3$, $NH(CH_2)_2C_6H_5$, Met-$R_3$, Leu-$R_3$, Ile-$R_3$, or Nle-$R_3$;
- $R_1$ represents hydrogen atom, linear or branched aliphatic chain having for 1 to 11 carbon atoms, benzyl or phenyl group;
- $R_2$ represents hydrogen atom or Tos, Dnp or Bzl group and
- $R_3$ represents an amino, hydroxy, methoxy or hydrazino group, and the pharmaceutically acceptable salts thereof.

**2.** A pharmaceutical composition comprising a peptide according to claim 1 or a pharmaceutically acceptable salt of such a peptide in admixture with a pharmaceutically acceptable diluent or carrier.

**3.** A process for the preparation of a peptide according to claim 1, the process comprising condensing amino acids and/or amino acid derivatives in the desired sequence and/or peptide fragments containing these amino acids or their derivatives in the desired sequence to give the desired peptide, the end carboxylic acid group being activated for the peptide linkage and the remaining groups being protected and deprotecting the resultant compound and/or converting the resultant peptide into a pharmaceutically acceptable salt thereof.

**4.** The use of the peptides according to claim 1 for the preparation of a pharmaceutical suitable in the therapy of human neoplasms.

**5.** The use of the peptides according to claim 1 for the preparation of a pharmaceutical useful as an antagonist versus effects induced by bombesin.

### Claims for the following Contracting State : ES

**1.** A process for the preparation of a peptide of the formula (I):

R-A-B-C-Trp-Ala-Val-X-Y-T-W (I)

wherein R represents a group of the formula $4\text{-}(ClCH_2CH_2)_2N\text{-}C_6H_4\text{-}CO\text{-}$, $3\text{-}(ClCH_2CH_2)_2N\text{-}C_6H_4\text{-}CO\text{-}$, $ClCH_2CH_2NHCO\text{-}$, $ClCH=CH\text{-}CO\text{-}$, $BrCH=CH\text{-}CO\text{-}$, $CH_2=CClCO\text{-}$, $CH_2=CBrCO\text{-}$(either cis or trans isomers),

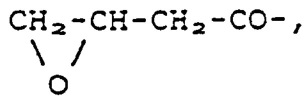

$HC \equiv C$-CO-, $ClCH_2CH_2N(NO)CO$-, or $ClCH_2CO$-$CH(R_1)NHCO(CH_2)_2CO$-;

- A represents a valence bond, or a Gly, Leu-Gly, Arg-Leu-Gly, Gln-Arg-Leu-Gly residue;
- B represents a valence bond or a Asn or Thr residue;
- C represents a Gln or His residue;
- X represents a Gly or ala residue;
- Y represents a valence bond or a His($R_2$), his($R_2$), Phe, phe, Ser, ser, Ala or ala residue;
- T represents a valence bond or a Leu, leu, Phe or phe residue;
- W represents a group of the formula OH, $NH_2$, $NH(CH_2)_4CH_3$, $NH(CH_2)_2C_6H_5$, Met-$R_3$, Leu-$R_3$, Ile-$R_3$, or Nle-$R_3$;
- $R_1$ represents hydrogen atom, linear or branched aliphatic chain having for 1 to 11 carbon atoms, benzyl or phenyl group;
- $R_2$ represents hydrogen atom or Tos, Dnp or Bzl group and
- $R_3$ represents an amino, hydroxy, methoxy or hydrazino group, and the pharmaceutically acceptable salts thereof

and the pharmaceutically acceptable salts thereof, said process comprising condensing amino acids and/or amino acid derivatives in the desired sequence and/or peptide fragments containing these amino acids or their derivatives in the desired sequence to give the desired peptide.

**2.** A process for the preparation of a peptide as defined in claim 1, said process further comprising that the end carboxylic acid group is activated for the peptide linkage and the remaining groups are protected and then deprotecting the resultant compound and/or converting the resultant peptide into a pharmaceutically acceptable salt thereof.

**3.** A process for the preparation of a pharmaceutical composition comprising a peptide as defined in claim 1 or a pharmaceutically acceptable salt of such a peptide, characterized by formulating the active ingredient as defined in claim 1 with a pharmaceutically acceptable diluent or carrier.

**4.** The process according to claim 3 for the preparation of a pharmaceutical suitable in the therapy of human neoplasms.

**5.** The process according to claim 3 for the preparation of a pharmaceutical useful as an antagonist versus effects induced by bombesin.

**Patentansprüche**

**Patentansprüche für folgende Vertragsstaaten : AT, BE, CH, DE, DK, FR, GB, IT, LI, LU, NL, SE**

**1.** Peptid der Formel I und pharmazeutisch verträgliche Salze davon:

R - A - B - C - Trp - Ala - Val - X - Y - T - W (I)

worin

- R eine Gruppe der Formel 4-$(ClCH_2CH_2)_2N$-$C_6H_4$-CO-, 3-$(ClCH_2CH_2)_2N$-$C_6H_4$-CO-, $ClCH_2CH_2NHCO$-, ClCH = CH-CO-, BrCH = CH-CO-, $CH_2$ = CClCO-, $CH_2$ = CBrCO- (entweder cis oder trans-Isomer)

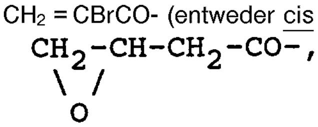

$CH \equiv C$-CO-, $ClCH_2CH_2N(NO)CO$-, oder $ClCH_2CO$-$CH(R_1)NHCO(CH_2)_2CO$- bedeutet;

- A eine Bindung oder einen Rest Gly, Leu-Gly, Arg-Leu-Gly oder Gln-Arg-Leu-Gly bedeutet;
- B eine Bindung oder einen Rest Asn oder Thr bedeutet;
- C einen Rest Gln oder His bedeutet;

X einen Rest Gly oder ala bedeutet;
Y eine Bindung oder einen Rest His($R_2$), his($R_2$), Phe, phe, Ser, ser, Ala oder ala bedeutet;
T eine Bindung oder einen Rest Leu, leu, Phe oder phe bedeutet;
W eine Gruppe der Formel OH, $NH_2$, $NH(CH_2)_4CH_3$, $NH(CH_2)_2C_6H_5$, Met-$R_3$, Leu-$R_3$, Ile-$R_3$ oder Nle-$R_3$ bedeutet;
$R_1$ ein Wasserstoffatom, eine lineare oder verzweigte aliphatische Kette mit 1 bis 11 Kohlenstoffatomen, eine Benzylgruppe oder eine Phenylgruppe bedeutet;
$R_2$ ein Wasserstoffatom oder eine Tos-, Dnp- oder Bzl-Gruppe bedeutet; und
$R_3$ eine Amino-, Hydroxy-, Methoxy- oder Hydrazin-Gruppe bedeutet.

**2.** Pharmazeutische Zusammensetzung, umfassend ein Peptid nach Anspruch 1 oder ein pharmazeutisch verträgliches Salz eines derartigen Peptids im Gemisch mit einem pharmazeutisch verträglichen Träger oder Verdünnungsmittel.

**3.** Verfahren zur Herstellung eines Peptids nach Anspruch 1, wobei das Verfahren das Kondensieren von Aminosäuren und/oder Aminosäurederivaten in der gewünschten Sequenz und/oder Peptidfragmenten, die diese Aminosäuren oder ihre Derivate in der gewünschten Sequenz enthalten, unter Bildung des gewünschten Peptids, wobei die endständige Carboxylgruppe für die Peptidverknüpfung aktiviert ist und die restlichen Gruppen geschützt sind, und das Entfernen der Schutzgruppen von der erhaltenen Verbindung und/oder die Umwandlung des erhaltenen Peptids in ein pharmazeutisch verträgliches Salz davon umfaßt.

**4.** Verwendung der Peptide nach Anspruch 1 zur Herstellung eines Arzneimittels, das zur Therapie von menschlichen Neoplasmen geeignet ist.

**5.** Verwendung der Peptide nach Anspruch 1 zur Herstellung eines Arzneimittels, das als Antagonist gegen die von Bombesin induzierten Wirkungen geeignet ist.

**Patentansprüche für folgenden Vertragsstaat : ES**

**1.** Verfahren zur Herstellung eines Peptids der Formel I und pharmazeutisch verträglicher Salze davon:

R - A - B - C - Trp - Ala - Val - X - Y - T - W (I)

worin
R eine Gruppe der Formel
4-$(ClCH_2CH_2)_2N$-$C_6H_4$-CO-, 3-$(ClCH_2CH_2)_2N$-$C_6H_4$-CO-, $ClCH_2CH_2NHCO$-, ClCH = CH-CO-, BrCH = CH-CO-, $CH_2$ = CClCO-, $CH_2$ = CBrCO- (entweder cis oder trans-Isomer)

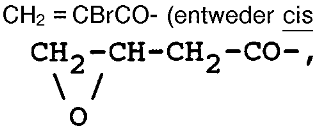

CH ≡ C-CO-, $ClCH_2CH_2N(NO)CO$-, oder $ClCH_2CO$-CH($R_1$)$NHCO(CH_2)_2CO$- bedeutet;
A eine Bindung oder einen Rest Gly, Leu-Gly, Arg-Leu-Gly oder Gln-Arg-Leu-Gly bedeutet;
B eine Bindung oder einen Rest Asn oder Thr bedeutet;
C einen Rest Gln oder His bedeutet;
X einen Rest Gly oder ala bedeutet;
Y eine Bindung oder einen Rest His($R_2$), his($R_2$), Phe, phe, Ser, ser, Ala oder ala bedeutet;
T eine Bindung oder einen Rest Leu, leu, Phe oder phe bedeutet;
W eine Gruppe der Formel OH, $NH_2$, $NH(CH_2)_4CH_3$, $NH(CH_2)_2C_6H_5$, Met-$R_3$, Leu-$R_3$, Ile-$R_3$ oder Nle-$R_3$ bedeutet;
$R_1$ ein Wasserstoffatom, eine lineare oder verzweigte aliphatische Kette mit 1 bis 11 Kohlenstoffatomen, eine Benzylgruppe oder eine Phenylgruppe bedeutet;
$R_2$ ein Wasserstoffatom oder eine Tos-, Dnp- oder Bzl-Gruppe bedeutet; und
$R_3$ eine Amino-, Hydroxy-, Methoxy- oder Hydrazin-Gruppe bedeutet,
wobei das Verfahren das Kondensieren von Aminosäuren und/oder Aminosäurederivaten in der gewünschten Sequenz und/oder Peptidfragmenten, die diese Aminosäuren oder ihre Derivate in der

gewünschten Sequenz enthalten, unter Bildung des gewünschten Peptids umfaßt.

2. Verfahren zur Herstellung eines Peptids, wie es in Anspruch 1 definiert ist, wobei das Verfahren ferner umfaßt, daß die endständige Carboxylgruppe für die Peptidverknüpfung aktiviert ist und die restlichen Gruppen geschützt sind und daß die Schutzgruppen von der erhaltenen Verbindung entfernt und/oder das erhaltene Peptid in ein pharmazeutisch verträgliches Salz davon umgewandelt wird.

3. Verfahren zur Herstellung einer pharmazeutischen Zusammensetzung, umfassend ein Peptid, wie es in Anspruch 1 definiert ist, oder ein pharmazeutisch verträgliches Salz eines derartigen Peptids, gekennzeichnet durch Formulieren des Wirkstoffs, wie er in Anspruch 1 definiert ist, mit einem pharmazeutisch verträglichen Träger oder Verdünnungsmittel.

4. Verfahren nach Anspruch 3 zur Herstellung eines Arzneimittels, das zur Therapie von menschlichen Neoplasmen geeignet ist.

5. Verfahren nach Anspruch 3 zur Herstellung eines Arzneimittels, das als Antagonist gegen die von Bombesin induzierten Wirkung geeignet ist.

**Revendications**
**Revendications pour les Etats contractants suivants : AT, BE, CH, DE, DK, FR, GB, IT, LI, LU, NL, SE**

1. Peptide représenté par la formule (I) :

R-A-B-C-Trp-Ala-Val-X-Y-T-W (I)

dans laquelle :
- R représente un groupe de la formule :
  4-$(ClCH_2CH_2)_2N$-$C_6H_4$-CO, 3-$(ClCH_2CH_2)_2N$-$C_6H_4$-CO-, $ClCH_2CH_2NHCO$-, ClCH = CH-CO-, BrCH = CH-CO-, $CH_2$ = CClCO-, $CH_2$ = CBrCO- (isomères soit cis soit trans),
  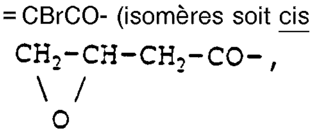
  HC≡C-CO-, $ClCH_2CH_2N(NO)CO$-, ou $ClCH_2CO$-$CH(R_1)NHCO(CH_2)_2CO$- ;
- A représente une liaison de valence, ou un reste Gly, Leu-Gly, Arg-Leu-Gly, Gln-Arg-Leu-Gly ;
- B représente une liaison de valence ou un reste Asn ou Thr ;
- C représente un reste Gln ou His ;
- X représente un reste Gly ou ala ;
- Y représente une liaison de valence ou un reste His($R_2$), his($R_2$), Phe, phe, Ser, ser, Ala ou ala ;
- T représente une liaison de valence ou un reste Leu, leu, Phe ou phe ;
- W représente un groupe de la formule OH, $NH_2$, $NH(CH_2)_4CH_3$, $NH(CH_2)_2C_6H_5$, Met-$R_3$, Leu-$R_3$, Ile-$R_3$, ou Nle-$R_3$ ;
- $R_1$ représente un atome d'hydrogène, une chaîne aliphatique linéaire ou ramifiée ayant de 1 à 11 atomes de carbone, un groupe benzyle ou phényle ;
- $R_2$ représente un atome d'hydrogène ou un groupe Tos, Dnp ou Bzl ; et
- $R_3$ représente un groupe amino, hydroxy, méthoxy ou hydrazino, et

ses sels pharmaceutiquement acceptables.

2. Composition pharmaceutique comprenant un peptide tel que défini à la revendication 1, ou un sel pharmaceutiquement acceptable d'un tel peptide, en mélange avec un diluant ou support pharmaceutiquement acceptable.

3. Procédé de préparation d'un peptide tel que défini à la revendication 1, le procédé comprenant la condensation d'acides aminés et/ou de dérivés d'acides aminés suivant la séquence désirée et/ou de fragments peptidiques contenant ces acides aminés ou leurs dérivés suivant la séquence désirée pour donner le peptide désiré, le groupe acide carboxylique terminal étant activé pour la liaison peptidique et les groupes restants étant protégés et, la déprotection du composé résultant et/ou la conversion du

peptide résultant en un sel pharmaceutiquement acceptable de celui-ci.

**4.** Utilisation des peptides tels que définis à la revendication 1 pour la préparation d'un produit pharmaceutique approprié dans la thérapie de néoplasmes humains.

**5.** Utilisation des peptides tels que définis à la revendication 1 pour la préparation d'un produit pharmaceutique utile en tant qu'antagoniste vis-à-vis des effets induits par la bombésine.

**Revendications pour l'Etat contractant suivant : ES**

**1.** Procédé de préparation d'un peptide représenté par la formule (I) :

R-A-B-C-Trp-Ala-Val-X-Y-T-W (I)

dans laquelle :

- R représente un groupe de la formule :
  4-$(ClCH_2CH_2)_2N$-$C_6H_4$-CO-, 3-$(ClCH_2CH_2)_2N$-$C_6H_4$-CO-, $ClCH_2CH_2NHCO$-, ClCH = CH-CO-, BrCH = CH-CO-, $CH_2$ = CClCO-, $CH_2$ = CBrCO- (isomères soit cis soit trans),

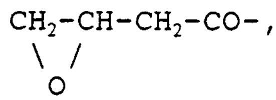

  HC≡C-CO-, $ClCH_2CH_2N(NO)CO$-, ou $ClCH_2CO$-$CH(R_1)NHCO(CH_2)_2CO$- ;
- A représente une liaison de valence, ou un reste Gly, Leu-Gly, Arg-Leu-Gly, Gln-Arg-Leu-Gly ;
- B représente une liaison de valence ou un reste Asn ou Thr ;
- C représente un reste Gln ou His ;
- X représente un reste Gly ou ala ;
- Y représente une liaison de valence ou un reste His($R_2$), his($R_2$), Phe, phe, Ser, ser, Ala ou ala ;
- T représente une liaison de valence ou un reste Leu, leu, Phe ou phe ;
- W représente un groupe de la formule OH, $NH_2$, $NH(CH_2)_4CH_3$, $NH(CH_2)_2C_6H_5$, Met-$R_3$, Leu-$R_3$, Ile-$R_3$, ou Nle-$R_3$ ;
- $R_1$ représente un atome d'hydrogène, une chaîne aliphatique, linéaire ou ramifiée, ayant de 1 à 11 atomes de carbone, un groupe benzyle ou phényle ;
- $R_2$ représente un atome d'hydrogène ou un groupe Tos, Dnp ou Bzl ; et
- $R_3$ représente un groupe amino, hydroxy, méthoxy ou hydrazino,

et de ses sels pharmaceutiquement acceptables,
ledit procédé comprenant la condensation d'acides aminés et/ou de dérivés d'acides aminés suivant la séquence désirée et/ou de fragments peptidiques contenant ces acides aminés ou leurs dérivés suivant la séquence désirée, pour donner le peptide désiré.

**2.** Procédé de préparation d'un peptide selon la revendication 1, ledit procédé comprenant en outre le fait que le groupe acide carboxylique terminal est activé pour la liaison peptidique et les groupes restants sont protégés, puis la déprotection du composé résultant et/ou la conversion du peptide résultant en un sel pharmaceutiquement acceptable de celui-ci.

**3.** Procédé de préparation d'une composition pharmaceutique comprenant un peptide tel que défini à la revendication 1 ou un sel pharmaceutiquement acceptable d'un tel peptide, caractérisé par le fait que l'on formule l'ingrédient actif tel que défini à la revendication 1 avec un diluant ou support pharmaceutiquement acceptable.

**4.** Procédé selon la revendication 3 pour la préparation d'un produit pharmaceutique approprié pour la thérapie de néoplasmes humains.

**5.** Procédé selon la revendication 3 pour la préparation d'un produit pharmaceutique utile comme antagoniste vis-à-vis des effets induits par la bombésine.